# EUROPEAN PATENT APPLICATION

(11) **EP 4 144 325 A1**
(43) Date of publication of application: **08.03.2023**
(21) Application number: 21804384.2
(22) Date of filing: 13.05.2021
(51) Int. Cl.: A61C 19/04, A61C 9/00, G06T 17/00

(54) **DENTAL SCANNING METHOD AND APPARATUS, COMPUTER DEVICE AND COMPUTER READABLE STORAGE MEDIUM**

(30) Priority: 14.05.2020 CN 202010406072
(71) Applicant: Shining 3D Tech Co., Ltd., Hangzhou, Zhejiang 311258 (CN)
(72) Inventor: ZHAO, Guanglin, Hangzhou, Zhejiang 311258 (CN); LIN, Zhongwei, Hangzhou, Zhejiang 311258 (CN); HU, Jun, Hangzhou, Zhejiang 311258 (CN); XUAN, Yizhou, Hangzhou, Zhejiang 311258 (CN)
(74) Representative: Grünecker Patent- und Rechtsanwälte PartG mbB
(86) International application number: PCT/CN2021/093687
(87) International publication number: WO 2021/228207

(57) **Abstract**

The present disclosure discloses a dental scanning method and device, computer device, and a non-transitory storage medium. The method includes: scanning a dental model according to a preset scanning path to generate a first mesh model; inputting the first mesh model into a preset dental identification model for identification to obtain dental data; searching tooth cavity data from the dental data according to a preset cavity seeking algorithm; and performing supplementary scanning on the dental model according to the tooth cavity data to generate a dentofacial mesh model. According to the dental scanning method provided by the embodiment of the disclosure, by performing intelligent supplementary scanning on the dental model, the problems of low efficiency and high operation difficulty of supplementary scanning of the tooth model are solved, and the quality of the dentofacial mesh model is improved.

## Description

The disclosure claims the priority of the Chinese patent application submitted to the Chinese Patent Office on May 14, 2020, with the application number of 202010406072.1 and titled "Dental Scanning Method and Device, System, and Non-transitory Storage Medium", of which the content is entirely incorporated herein by reference.

### Technical Field

The disclosure relates to the technical field of three-dimensional scanning, and in particular to a dental scanning method and device, a computer device, and a non-transitory storage medium.

### Background Art

In a case that a dental clinic technician or doctor scans a tooth model with a desktop three-dimensional scanner, a clearer model can be obtained only after repeated supplementary scanning because deep seams of the tooth model are too deep. When scanning the tooth model, scanner software will scan according to the specific fixed paths of different models. After the scanning is completed, in a case that some areas cannot be scanned completely, an operator needs to adjust the viewing angle to the area that has not been scanned for manual supplementary scanning, and manually supplementary scanning will be repeatedly performed until the model scanning is completed. This scanning process needs a lot of time and labor costs, is lower in efficiency of supplementary scanning, easily caused the problems of failure in data supplementary scanning at certain positions and in finding of correct supplementary scanning angles of the deep seams and undercuts, requiring too much technology and experience, and requiring too many in times of supplementary scanning and too slow in efficiency and the like, is inconvenient to be used, and increases the time and difficulty for operation; and the supplementary scanning requires certain skills and has certain difficulty for operation, so that when the operator has no relevant experience, it is impossible to accurately scan the complete picture of the tooth model.

Currently, no effective solutions have been proposed for the low efficiency and difficult operation of the supplementary scanning of the tooth model in the related art.

### Summary of the Invention

The embodiments of the disclosure provide a dental scanning method and device, a system, a computer device, and a non-transitory storage medium, so as to at least solve the problems of low efficiency and difficult operation of the supplementary scanning of the tooth model in the related art.

In a first aspect, the embodiments of the disclosure provide:
A dental scanning method, which includes: a dental model is scanned according to a preset scanning path to generate a first mesh model; the first mesh model is input into a preset dental identification model for identification to obtain dental data; tooth cavity data is searched from the dental data according to a preset cavity seeking algorithm; and supplementary scanning is performed on the dental model according to the tooth cavity data to generate a dentofacial mesh model.

In some embodiments, the operation that supplementary scanning is performed on the dental model according to the tooth cavity data to generate the dentofacial mesh model includes:
a supplementary scanning path is generated according to the tooth cavity data;
the dental model is scanned according to the supplementary scanning path to obtain supplementary scanning data; and
the dentofacial mesh model is generated according to the supplementary scanning data and the first mesh model.

In some embodiments, the operation that the dentofacial mesh model is generated according to the supplementary scanning data and the first mesh model includes:
a supplementary scanning gain is calculated according to the supplementary scanning data; and
in a case that the supplementary scanning gain is less than a preset threshold, the dentofacial mesh model is generated according to the supplementary scanning data and the first mesh model.

In some of the embodiments, the operation the supplementary scanning gain is calculated according to the supplementary scanning data and the first mesh model includes:
in a case that the supplementary scanning gain is greater than the preset threshold, the first mesh model is updated according to the supplementary scanning data to generate an iterative mesh model;
an iterative scanning path is generated according to the iterative mesh model; and
the dental model is scanned according to the iterative scanning path to obtain iterative scanning data until the supplementary scanning gain of the iterative scanning data is less than the preset threshold, and the dentofacial mesh model is generated according to the iterative scanning data and the iterative mesh model.

In some of the embodiments, the supplementary scanning path includes a first scanning path and a second scanning path; the operation that the dental model is scanning according to the supplementary scanning path to obtain supplementary scanning data includes: the dental model is scanned according to the first scanning path to obtain first scanning data;
path calculation is performed according to the first scanning data to obtain the second scanning path;
the dental model is scanned according to the second scanning path to obtain second scanning data; and the first scanning data and the second scanning data are taken as the supplementary scanning data.

In some of the embodiments, before the operation that the first mesh model is input into the preset dental identification model for identification to obtain dental data, the method includes: a neural network is established, and the first mesh model is used as a training set to train a neural network model to obtain the dental identification model, an input of the dental identification model is the first mesh model, and an output is the dental data in the first mesh model.

In some of the embodiments, the operation that tooth cavity data is searched from the dental data according to a preset cavity seeking algorithm includes: the dental data is analyzed to obtain an abutment tooth area; the tooth cavity data is searched according to the preset cavity seeking algorithm based on the abutment tooth area; and the corresponding tooth cavities are identified in the first mesh model according to the tooth cavity data.

In a second aspect, the embodiments of the disclosure provide a dental scanning device, which includes: a scanning component, configured to scan a dental model according to a preset scanning path to generate a first mesh model; a recognition component, configured to input the first mesh model into a preset dental identification model for identification to obtain dental data; a cavity searching component, configured to search the dental data for tooth cavities according to a preset cavity seeking algorithm to obtain tooth cavity data; and a supplementary scanning component, configured to perform supplementary scanning on the dental model according to the tooth cavity data to generate a dentofacial mesh model.

In a third aspect, the embodiments of the disclosure provide computer device, which includes a memory, a processor, and a computer program stored on the memory and capable of running on the processor, and when the processor executes the computer program, the dental scanning method as described in the first aspect is implemented.

In a fourth aspect, the embodiments of the disclosure provide a non-transitory storage medium, which stores a computer program, and when being performed by the processor, the computer program implements the dental scanning method as described in the first aspect.

Compared with the related art, according to the dental scanning method provided by the embodiments of the disclosure, by performing intelligent supplementary scanning on the dental model, the problems of low efficiency and high operation difficulty of supplementary scanning of the tooth model are solved, and the quality of the dentofacial mesh model is improved.

The details of one or more embodiments of the disclosure are presented in the following accompanying drawings and descriptions to make other features, objectives and advantages of the disclosure more concise and understandable.

### Brief Description of the Drawings

The accompanying drawings described herein serve to provide a further understanding of the disclosure, and constitute a part of the disclosure. The exemplary embodiments of the disclosure and the descriptions thereof serve to explain the disclosure, but do not unduly limit the disclosure. In figures:
FIG. 1 is a flowchart of a dental scanning method according to an embodiment of the disclosure;
FIG. 2 is a flowchart of path calculation according to an embodiment of the disclosure;
FIG. 3 is a flowchart of another dental scanning method according to an embodiment of the disclosure;
FIG. 4 is a structural block diagram of a dental scanning device according to an embodiment of the disclosure; and
FIG. 5 is a schematic diagram of a hardware structure of dental scanning device according to an embodiment of the disclosure.

### Detailed Description of the Invention

In order to make the objectives, technical solutions, and advantages of the disclosure clearer, the disclosure is described and illustrated below in conjunction with the accompanying drawings and embodiments. It is to be understood that specific embodiments described herein are only adopted to explain the disclosure and not intended to limit the disclosure. All other embodiments obtained by those of ordinary skill in the art based on the embodiments of the disclosure without creative efforts fall within the protection scope of the disclosure.

Obviously, the accompanying drawings in the following description are only some examples or embodiments of the disclosure. For those of ordinary skill in the art, the disclosure can also be applied to other similar scenarios according to these accompanying drawings without creative efforts. In addition, it can also be understood that although the efforts made in this development process may be complicated and lengthy, for those of ordinary skill in the art related to the content disclosed in the disclosure, changes in design, manufacturing or production based on the technical content disclosed in the disclosure are only conventional technical means, and should not be understood as insufficient content disclosed in the disclosure.

The reference to "embodiments" in the disclosure means that specific features, structures or characteristics described in conjunction with the embodiments may be included in at least one embodiment of the disclosure. The appearance of the phrase in various places in the description does not necessarily refer to the same embodiment, nor is it an independent or alternative embodiment mutually exclusive with other embodiments. It is understood explicitly and implicitly by those of ordinary skill in the art that the embodiments described in the disclosure can be combined with other embodiments without conflict.

Unless otherwise defined, the technical terms or scientific terms involved in the disclosure shall be in the usual sense as understood by people with general skills in the technical field to which the disclosure belongs. The words "a", "an", "one", "the" and the like referred to in the disclosure do not imply a quantitative limit, and may denote the singular or plural. The terms "comprise", "include", "have" and any variation thereof referred to in the disclosure are intended to cover non-exclusive inclusion; for example, a process, method, system, product or device that includes a series of steps or components (units) is not limited to the listed steps or units, but can also include steps or units that are not listed, or can also include other steps or units inherent to these processes, methods, products or device. The words "connect", "be connected to", "couple" and the like referred to in the disclosure are not limited to physical or mechanical connection, but can include electrical connection, whether direct or indirect. The "plurality" referred to in the disclosure refers to two or more. "And/or" describes the association relationship of associated objects, which means that there can be three kinds of relationships. For example, "A and/or B" may indicate that there are three situations: A alone, A and B together, and B alone. The character "/" generally indicates that the associated objects are in an "or" relationship. The terms "first", "second", "third" and the like referred to in the disclosure merely distinguish similar objects, and do not represent a specific ordering of the objects.

The various technologies described in the disclosure can be used in various three-dimensional scanners. Three-dimensional scanners are also known as 3D scanners, are high-tech products that integrate optical, mechanical, electrical and computer technologies, and are mainly used for obtaining the three-dimensional coordinates of outer surfaces of objects and three-dimensional digital models of the objects. In the embodiment, the three-dimensional scanner is a desktop three-dimensional scanner.

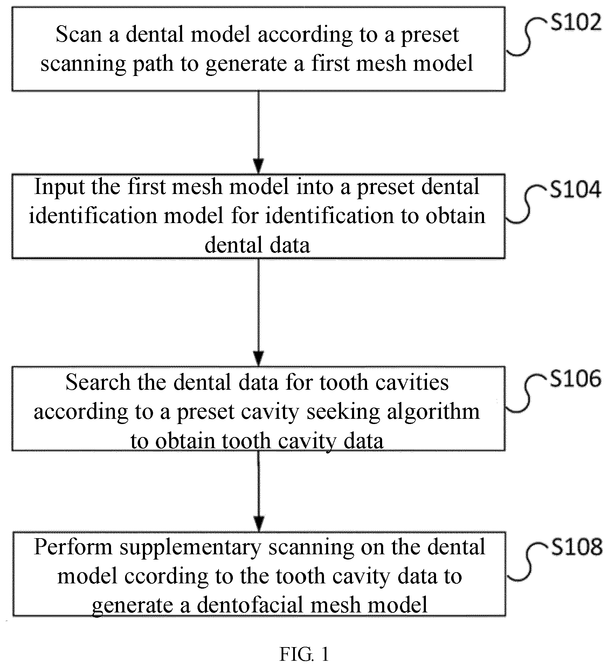

The embodiment also provides a dental scanning method. FIG. 1 is a flowchart of a dental scanning method according to an embodiment of the disclosure; and as shown in FIG. 1, the process includes the following steps:
Step S102, a dental model is scanned according to a preset scanning path to generate a first mesh model.

Wherein the dental model is a tooth model with an imparted model, a scanning rod or an implant abutment. The preset scanning path is a path pre-stored in a three-dimensional scanner and used for scanning the dental model.

Alternatively, the dental model is scanned according to a fixed scanning path, and the obtained data is optimized and de-curled while scanning. The first mesh model is generated by using an incremental rapid processing method according to the optimized scanning data. In some embodiment, the data optimization is to perform data matrix alignment by comparing the coordinates of all the data, and de-curling is to remove warped edges of model edges in the scanning data and the data with poorer models.

Step S104, the first mesh model is input into a preset dental identification model for identification to obtain dental data.

Alternatively, the first mesh model is input into the preset dental identification model, an artificial intelligence algorithm is adopted to automatically identify the first mesh model, and the dental data is identified by comparing and analyzing all areas of the first mesh model. The dental data includes common teeth, an abutment tooth area, a scanning rod, an abutment and other types of data. Abutment teeth, also known as bridge abutment teeth, are part of the components of fixed partial denture and are teeth used for installing retainers. The abutment tooth area is the area where the abutment teeth are located. The scanning rod is a standard body accessory used by the three-dimensional scanner to scan the dental model. The scanning rod can be inserted into the dental jaw, and the position of an implant can be better positioned by scanning the standard scanning rod on the jaw. The abutment is a non-screw-retained customizable scanning rod accessory.

Before the first mesh model is input into the preset dental identification model for identification to obtain the dental data, the method includes: a neural network is established, and the first mesh model is used as a training set to train a neural network model to obtain the dental identification model, an input of the dental identification model is the first mesh model, and an output is the dental data in the first mesh model.

Alternatively, the first mesh model is used as the training set to train the neural network model. The neural network model identifies the first mesh model by learning the internal rules and representation levels of sample data. After a large amount of deep learning training, the dental identification model is generated. Further, the dental identification model can also perform deep learning through a process of subsequent identification of the first mesh model.

Step S106: the dental data is searched for tooth cavities according to a preset cavity seeking algorithm to obtain tooth cavity data.

The tooth cavities are holes in the abutment tooth area of a dentofacial mesh model generated by scanning. Because the dentofacial model has undercuts or shadows, or the scanning path fails to cover the deep holes, holes present in the scanned mesh model.

Alternatively, the tooth cavities in the dental data are found through the preset cavity seeking algorithm, thus obtaining the tooth cavity data. The tooth cavity data includes tooth cavity contours and tooth cavity depths. Further, the dental data is analyzed to obtain the abutment tooth area; the tooth cavities are searched according to the preset cavity seeking algorithm based on the abutment tooth area to obtain the tooth cavity data; and the corresponding tooth cavities in the first mesh model are identified according to the tooth cavity data. In the embodiment, the tooth cavity data and the first mesh model are input into the same scene for display, and edges of the tooth cavities in the first mesh model are marked with red. An operator can also manually adjust the marks of the tooth cavities to make the marked data more suitable for the actual situation.

Step S108, supplementary scanning is performed on the dental model according to the tooth cavity data to generate the dentofacial mesh model.

Alternatively, a supplementary scanning path is generated according to the tooth cavity data. The dental model is scanned according to the supplementary scanning path to obtain supplementary scanning data. The dentofacial mesh model is generated according to the supplementary scanning data and the first mesh model. Further, meshes in the first mesh model are merged and optimized, the first mesh model is identified according to the tooth cavity data, and areas that need to be subjected to supplementary scanning are marked. Path calculation is performed on the areas that need to be subjected to supplementary scanning in the first mesh model to generate a supplementary scanning path with a maximum data profit value. The dental model is scanned according to the supplementary scanning path to obtain supplementary scanning data. The first mesh model is iteratively updated according to the supplementary scanning data to generate the dentofacial mesh model. In the embodiment, the first mesh model is identified according to the tooth cavity data, and the areas that need to be subjected to supplementary scanning are marked. The operator can also actively mark the areas that need to be subjected to supplementary scanning to merge two of the areas, thus generating an area determined for supplementary scanning. Path calculation is performed according to the area determined for supplementary scanning to generate the supplementary scanning path with the maximum data profit value.

The step that the dentofacial mesh model is generated according to the supplementary scanning data and the first mesh model includes: a supplementary scanning gain is calculated according to the supplementary scanning data; and in a case that the supplementary scanning gain is less than a preset threshold, the dentofacial mesh model is generated according to the supplementary scanning data and the first mesh model.

Alternatively, in a case that the supplementary scanning gain is greater than the preset threshold, the first mesh model is updated according to the supplementary scanning data to generate an iterative mesh model; an iterative scanning path is generated according to the iterative mesh model; and the dental model is scanned according to the iterative scanning path to obtain iterative scanning data until the supplementary scanning gain of the iterative scanning data is less than the preset threshold, and the dentofacial mesh model is generated according to the iterative scanning data and the iterative mesh model. In the embodiment, the first mesh model is updated according to the supplementary scanning data to generate the iterative mesh model: low-definition regions lower than a definition threshold in the iterative mesh model are obtained by calculation, the iterative scanning path is generated according to the low-definition regions, and the dental model is scanned according to the iterative scanning path to obtain the iterative scanning data. Repeat scanning is performed and the previous model is iterated until the supplementary scanning gain of the iterative scanning data is less than the preset threshold, and the dentofacial mesh model is generated according to the iterative scanning data and the iterative mesh model.

The step that the dental model is scanned according to the supplementary scanning path to obtain the supplementary scanning data includes: the dental model is scanned according to the first scanning path to obtain first scanning data; path calculation is performed according to the first scanning data to obtain a second scanning path; and the dental model is scanned according to the second scanning path to obtain second scanning data. The supplementary scanning path includes the first scanning path and the second scanning path.

Alternatively, the dental model is scanned according to the first scanning path, thus obtaining the first scanning data. Because the first scanning path cannot traverse all areas in the dental model, the areas that are not scanned by the first scanning data in the dental model are calculated, the second scanning path with the maximum data profit value is calculated according to the areas that are not scanned, and the dental model is scanned according to the second scanning path to obtain the second scanning data; and the first scanning data and the second scanning data are used as the supplementary scanning data. It can be understood that in a case that the first scanning path and the second scanning data do not traverse all the areas in the dental model, a third scanning path and a fourth scanning path may also be calculated, which will not be repeated here. Further, the scanning times are preset, and when the preset scanning times are reached, the first mesh model is updated according to the supplementary scanning data to generate the iterative mesh model; the iterative scanning path is generated according to the iterative mesh model; and the dental model is scanned according to the iterative scanning path to obtain the iterative scanning data. The dentofacial mesh model is generated according to the iterative scanning data and the iterative mesh model.

Through the above steps, the first mesh model is generated by scanning the dental model; the tooth cavity data in the first mesh model is identified, and the dental model is subjected to supplementary scanning according to the tooth cavity data, so that the dentofacial mesh model is generated. By performing intelligent supplementary scanning on the dental model, the problems of low efficiency and high operation difficulty of supplementary scanning of the tooth model are solved, and the quality of the dentofacial mesh model is improved.

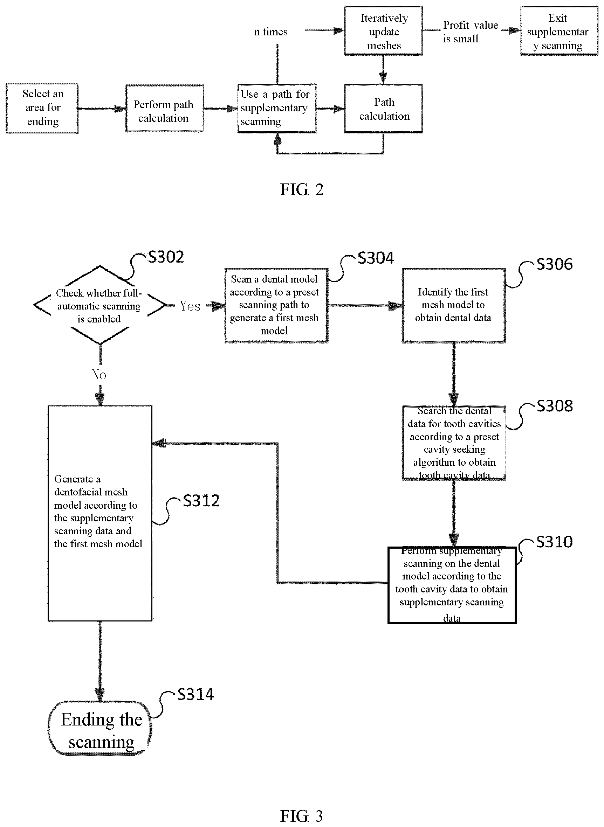

The embodiment also provides a path calculation method. As shown in FIG. 2, the process includes the following steps:
Areas that need to be subjected to supplementary scanning are selected and path calculation is performed according to the selected areas that need to be subjected to supplementary scanning to obtain a first scanning path. A dental model is scanned according to the first scanning path to obtain first scanning data. Path calculation is performed on the areas which are not scanned by the first scanning path in the areas that need to be subjected to supplementary scanning to obtain a second scanning path; and the dental model is scanned according to the second scanning path to obtain second scanning data. After N times of scanning, a first mesh model is iteratively updated according to the scanning data of multiple times of scanning to generate an iterative mesh model; an iterative scanning path is generated according to the iterative mesh model; and the dental model is scanned according to the iterative scanning path to obtain iterative scanning data until a supplementary scanning gain of the iterative scanning data is less than a preset threshold, and a dentofacial mesh model is generated according to the iterative scanning data and the iterative mesh model.

The embodiment also provides a dental scanning method. FIG. 3 is a flowchart of another dental scanning method according to an embodiment of the disclosure; and as shown in FIG. 3, the process includes the following steps:
Step S302, it is checked that whether full-automatic scanning is enabled.
Step S304, in a case that the full-automatic scanning is enabled; a dental model is scanned according to a preset scanning path to generate a first mesh model.
Step S306, the first mesh model is identified to obtain dental data.
Step S308, the dental data is searched for tooth cavities according to a preset cavity seeking algorithm to obtain tooth cavity data.
Step S310, supplementary scanning is performed on the dental model according to the tooth cavity data to obtain supplementary scanning data.
Step S312, a dentofacial mesh model is generated according to the supplementary scanning data and the first mesh model to complete the scanning.
Step S314, in a case that the full-automatic scanning is not enabled, the dental model is scanned directly to generate a corresponding dental mesh model to complete the scanning.

It should be noted that the steps in the process or shown in the flowchart of the drawings may be performed in a computer system, such as a set of computer-executable instructions. Moreover, although a logic sequence is shown in the flowchart, the shown or described steps may be performed in a sequence different from the sequence here under certain conditions.

The embodiment also provides a dental scanning device, which is used for implementing the above-mentioned embodiments and preferred implementations, and those that have been described will not be repeated. As used below, the terms "component", "unit", "sub-unit", and the like may be a combination of software and/or hardware that can realize predetermined functions. Although the device described in the following embodiments is preferably implemented by software, implementation by hardware or a combination of software and hardware is also possible and conceived.

It should be understood that although the steps of the flowcharts in FIGs. 1-3 are shown sequentially as indicated by the arrows, the steps are not necessarily performed sequentially as indicated by the arrows. Unless alternatively stated otherwise herein, the steps are not performed in a strict order of limitation, and the steps may be performed in other orders. Moreover, at least a portion of the steps in FIGs. 1-3 may include a plurality of sub-steps or a plurality of stages that are not necessarily performed at the same time, but may be performed at different times, and the sub-steps or stages may be not necessarily performed sequentially, but may be performed in turn or alternation with at least a portion of other steps or the sub-steps or stages of other steps.

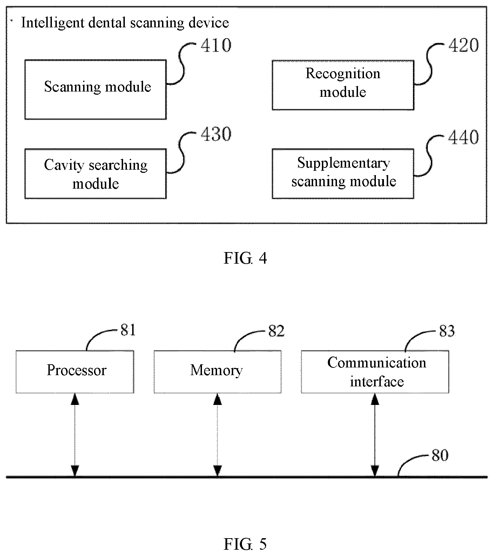

FIG. 4 is a structural block diagram of a dental scanning device according to an embodiment of the disclosure; and as shown in FIG. 4, the device includes: a scanning component 410, a recognition component 420, a cavity searching component 430, and a supplementary scanning component 440.

The scanning component 410 is configured to scan a dental model according to a preset scanning path to generate a first mesh model.

The recognition component 420 is configured to input the first mesh model into a preset dental identification model for identification to obtain dental data.

The cavity searching component 430 is configured to search the dental data for tooth cavities according to a preset cavity seeking algorithm to obtain tooth cavity data.

The supplementary scanning component 440 is configured to perform supplementary scanning on the dental model according to the tooth cavity data to generate a dentofacial mesh model.

The supplementary scanning component 440 is further configured to generate a supplementary scanning path according to the tooth cavity data, scan the dental model according to the supplementary scanning path to obtain supplementary scanning data, and generate the dentofacial mesh model according to the supplementary scanning data and the first mesh model.

The supplementary scanning component 440 is further configured to calculate a supplementary scanning gain according to the supplementary scanning data, and generate the dentofacial mesh model according to the supplementary scanning data and the first mesh model in a case that the supplementary scanning gain is less than a preset threshold.

The supplementary scanning component 440 is further configured to update the first mesh model according to the supplementary scanning data to generate an iterative mesh model in a case that the supplementary scanning gain is greater than the preset threshold, generate an iterative scanning path according to the iterative mesh model, scan the dental model according to the iterative scanning path to obtain iterative scanning data until the supplementary scanning gain of the iterative scanning data is less than the preset threshold, and generate the dentofacial mesh model according to the iterative scanning data and the iterative mesh model.

The supplementary scanning component 440 is further configured to scan the dental model according to a first scanning path to obtain first scanning data, perform path calculation according to the first scanning data to obtain a second scanning path, scan the dental model according to the second scanning path to obtain second scanning data, and take the first scanning data and the second scanning data as the supplementary scanning data.

The recognition component 420 is further configured to establish a neural network, and use the first mesh model as a training set to train a neural network model to obtain the dental identification model, an input of the dental identification model is the first mesh model, and an output is the dental data in the first mesh model.

The cavity searching component 430 is further configured to analyze the dental data to obtain an abutment tooth area, search the tooth cavities according to the preset cavity seeking algorithm based on the abutment tooth area to obtain the tooth cavity data, and identify the corresponding tooth cavities in the first mesh model according to the tooth cavity data.

It should be noted that each of the above components may be a functional component or a program component, and may be implemented by software or hardware. For the components implemented by the hardware, all the above-mentioned components may be located in the same processor; or all the above-mentioned components may also be located in different processors in a form of any combination.

In addition, the dental scanning method of the embodiment of the disclosure described in conjunction with FIG. 1 can be implemented by dental scanning device. FIG. 5 is a schematic diagram of a hardware structure of dental scanning device according to an embodiment of the disclosure.

The dental scanning device may include a processor 81 and a memory 82 storing computer program instructions.

Alternatively, the processor 81 may include a central processing unit (CPU), or an application specific integrated circuit (ASIC for short), or may be configured into one or more integrated circuits for implementing the embodiments of the disclosure.

The memory 82 may include a mass memory for data or instructions. For example, rather than as a limitation, the memory 82 may include a hard disk drive (HDD for short), a floppy disk drive, a solid state drive (SSD for short), a flash memory, an optical disk, a magneto-optical disk, a magnetic tape or a universal serial bus (USB for short) drive, or a combination of two or more thereof. Where appropriate, the memory 82 may include a removable or non-removable (or fixed) media. Where appropriate, the memory 82 may be positioned inside or outside a data processing apparatus. In a specific embodiment, the memory 82 is a non-volatile memory. In a specific embodiment, the memory 82 includes a read-only memory (ROM for short) and a random access memory (RAM for short). Where appropriate, the ROM can be a mask-programmed ROM, a programmable read-only memory (PROM for short), an erasable programmable read-only memory (EPROM for short), an electrically erasable programmable read-only memory (EEPROM for short), an electrically alterable read-only memory (EAROM for short), a flash memory, or a combination of two or more thereof. Where appropriate, the RAM can be a static random access memory (SRAM for short) or a dynamic random access memory (DRAM for short), and the DRAM can be a fast page mode dynamic random access memory (FPMDRAM for short), an extended data output dynamic random access memory (EDODRAM for short), a synchronous dynamic random access memory (SDRAM for short) and the like.

The memory 82 may be used for storing or caching various data files that need to be processed and/or used for communication, and possible computer program instructions performed by the processor 81.

The processor 81 reads and executes the computer program instructions stored in the memory 82 to implement any one of the dental scanning methods in the foregoing embodiments.

In some of the embodiments, the dental scanning device may further include a communication interface 83 and a bus 80. As shown in FIG. 5, the processor 81, the memory 82 and the communication interface 83 are connected by means of the bus 80 and complete the communication with each other.

The communication interface 83 is used for implementing communication between various components, devices, units and/or device in the embodiments of the disclosure. The communication interface 83 can also implement data communication with other components such as external device, image/data acquisition device, databases, external memories, and image/data processing workstations.

The bus 80 includes hardware, software, or both, and couples the components of the dental scanning device to each other. The bus 80 includes but is not limited to at least one of the following: a data bus, an address bus, a control bus, an expansion bus, and a local bus. For example, rather than as a limitation, the bus 80 may include an accelerated graphics port (AGP for short) or other graphics buses, an extended industry standard architecture (EISA for short) bus, a front side bus (FSB for short), a hyper transport (HT for short) interconnect, an industry standard architecture (ISA for short) bus, an InfiniBand interconnect, a low pin count (LPC for short) bus, a memory bus, a micro channel architecture (MCA for short) bus, a peripheral component interconnect (PCI for short) bus, a PCI-Express (PCI-X) bus, a serial advanced technology attachment (SATA for short) bus, a video electronics standards association local bus (VLB for short), or other suitable buses, or a combination of two or more thereof. Where appropriate, the bus 80 may include one or more buses. Although the embodiments of the disclosure describe and show specific buses, the disclosure considers any suitable bus or interconnect.

The dental scanning device can execute the dental scanning method in the embodiments of the disclosure based on the acquired three-dimensional data, thereby realizing the dental scanning method described in conjunction with FIG. 1.

In addition, in combination with the dental scanning method in the foregoing embodiments, the embodiment of the disclosure may provide a non-transitory storage medium for implementation. Computer program instructions are stored on the non-transitory storage medium; and when the computer program instructions are performed by the processor, any one of the dental scanning methods in the foregoing embodiments is implemented.

The technical features of the above embodiments may be combined arbitrarily. In order to simplify the description, not all possible combinations of the technical features in the above embodiments are completely described. However, as long as there is no conflict between these technical features, they should be considered to be within the scope of this description.

The foregoing embodiments only describe several implementations of the disclosure, and their description is relatively specific and detailed, but cannot therefore be understood as a limitation to the patent scope of the present disclosure. It should be noted that those of ordinary skill in the art may further make several variations and improvements without departing from the conception of the disclosure, and these variations and improvements all fall within the protection scope of the disclosure. Therefore, the patent protection scope of the disclosure should be subject to the appended claims.

### Industrial applicability

The solutions provided by the embodiments of the present disclosure can be applied to a process of dental scanning. The embodiments of the present disclosure solve the technical problems of low efficiency and high operation difficulty of supplementary scanning of a tooth model, and realize the technical effect of improving the quality of a dentofacial mesh model.

## Claims

1. A dental scanning method, comprising
scanning a dental model according to a preset scanning path to generate a first mesh model;
inputting the first mesh model into a preset dental identification model for identification to obtain dental data;
searching tooth cavity data from the dental data according to a preset cavity seeking algorithm; and
performing supplementary scanning on the dental model according to the tooth cavity data to generate a dentofacial mesh model.

2. The dental scanning method as claimed in claim 1, wherein the step of performing supplementary scanning on the dental model according to the tooth cavity data to generate the dentofacial mesh model comprises:
generating a supplementary scanning path according to the tooth cavity data;
scanning the dental model according to the supplementary scanning path to obtain supplementary scanning data; and
generating the dentofacial mesh model according to the supplementary scanning data and the first mesh model.

3. The dental scanning method as claimed in claim 2, wherein the step of generating the dentofacial mesh model according to the supplementary scanning data and the first mesh model comprises:
calculating a supplementary scanning gain according to the supplementary scanning data; and
in a case that the supplementary scanning gain is less than a preset threshold, generating the dentofacial mesh model according to the supplementary scanning data and the first mesh model.

4. The dental scanning method as claimed in claim 3, wherein the step of calculating the supplementary scanning gain according to the supplementary scanning data and the first mesh model comprises:
in a case that the supplementary scanning gain is greater than the preset threshold, updating the first mesh model according to the supplementary scanning data to generate an iterative mesh model;
generating an iterative scanning path according to the iterative mesh model; and
scanning the dental model according to the iterative scanning path to obtain iterative scanning data until the supplementary scanning gain of the iterative scanning data is less than the preset threshold, and generating the dentofacial mesh model according to the iterative scanning data and the iterative mesh model.

5. The dental scanning method as claimed in claim 2, wherein the step of scanning the dental model according to the supplementary scanning path to obtain supplementary scanning data comprises:
the supplementary scanning path comprising a first scanning path and a second scanning path;
scanning the dental model according to the first scanning path to obtain first scanning data;
performing path calculation according to the first scanning data to obtain the second scanning path;
scanning the dental model according to the second scanning path to obtain second scanning data; and
taking the first scanning data and the second scanning data as the supplementary scanning data.

6. The dental scanning method as claimed in claim 1, wherein before the step of inputting the first mesh model into the preset dental identification model for identification to obtain dental data, the method comprises:
establishing a neural network, and taking the first mesh model as a training set to train a neural network model to obtain the dental identification model, an input of the dental identification model is the first mesh model, and an output is the dental data in the first mesh model.

7. The dental scanning method as claimed in claim 1, wherein the step of searching the tooth cavity data from the dental data according to the preset cavity seeking algorithm comprises:
analyzing the dental data to obtain an abutment tooth area;
search the tooth cavity data according to the preset cavity seeking algorithm based on the abutment tooth area; and
identifying corresponding tooth cavities in the first mesh model according to the tooth cavity data.

8. A dental scanning device, comprising:
a scanning component, configured to scan a dental model according to a preset scanning path to generate a first mesh model;
a recognition component, configured to input the first mesh model into a preset dental identification model for identification to obtain dental data;
a cavity searching component, configured to search tooth cavity data from the dental data according to a preset cavity seeking algorithm; and
a supplementary scanning component, configured to perform supplementary scanning on the dental model according to the tooth cavity data to generate a dentofacial mesh model.

9. A computer device, comprising a memory, a processor, and a computer program stored on the memory and capable of running on the processor, wherein when the processor executes the computer program, the dental scanning method as claimed in anyone of claims 1 to 7 is implemented.

10. A computer-readable storage medium, storing a computer program, wherein when being performed by the processor, the computer program implements the dental scanning method as claimed in any one of claims 1 to 7.
